# EUROPÄISCHE PATENTANMELDUNG

(11) **EP 2 266 638 A2**
(43) Veröffentlichungstag der Anmeldung: **29.12.2010**
(21) Anmeldenummer: 10158590.9
(22) Anmeldetag: 31.03.2010
(51) Int. Cl.: A61L 31/02, A61L 31/14, A61L 31/16

(54) **Biokorrodierbares Implantat mit einer aktiven Beschichtung**

(30) Priorität: 25.06.2009 US 220219 P
(71) Anmelder: Biotronik VI Patent AG, 6341 Baar (CH)
(72) Erfinder: Borck, Alexander, 91086, Aurachtal (DE)
(74) Vertreter: Lindner-Vogt, Karin L.

(57) **Zusammenfassung**

Die Erfindung betrifft ein Implantat mit einem Grundkörper aus einem biokorrodicrbaren Werkstoff und mit einer aktiven Beschichtung oder Füllung einer Kavität bestehend aus oder enthaltend die Komponenten
a) zumindest eine pharmazeutisch aktive Substanz; und
b) einen Hilfsstoff zur Verbesserung der Permeabilität der mindestens einen pharmazeutisch aktiven Substanz.

## Beschreibung

Die Erfindung betrifft ein Implantat aus einem biokorrodierbaren Werkstoff mit einer aktiven Beschichtung oder Kavitätenfüllung, die wenigstens eine pharmazeutische Substanz enthält.

### Hintergrund der Erfindung

Medizinische Implantate unterschiedlichster Zweckbestimmung sind in großer Vielfalt aus dem Stand der Technik bekannt. Häufig ist nur ein zeitweiliger Verbleib des Implantats im Körper zur Erfüllung des medizinischen Zweckes erforderlich. Implantate aus permanenten Werkstoffen, also Werkstoffen, die im Körper nicht abgebaut werden, sind häufig wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht nun darin, das Implantat ganz oder in Teilen aus einem biokorrodierbaren Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert, wobei geringe Rückstände tolerierbar sind.

Biokorrodierbare Werkstoffe wurden unter anderem auf Basis von Polymeren synthetischer Natur oder natürlichen Ursprungs entwickelt. Die Materialeigenschaften, aber auch teils die Biokompatibilität der Abbauprodukte der Polymere, limitieren den Einsatz jedoch deutlich. So müssen beispielsweise orthopädische Implantate häufig hohen mechanischen Beanspruchungen standhalten und vaskuläre Implantate, z.B. Stents, je nach Design sehr speziellen Anforderungen an E-Modul, Bruchfestigkeit und Formbarkeit genügen.

In DE 197 31 021 A1 wird vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stents, eignet.

Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen. Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Es ist ferner bekannt, auf dem Stent eine pharmazeutisch aktive Substanz (Wirkstoff) bereitzustellen, die insbesondere das Restenoserisiko senken und den Heilungsverlauf unterstützen soll. Der Wirkstoff kann in Reinform oder eingebettet in eine Trägermatrix als Beschichtung aufgetragen oder in Kavitäten des Implantats bereitgestellt werden. Beispiele geeigneter Wirkstoffe umfassen Sirolimus und Paclitaxel.

Ein Problem bei der Optimierung wirkstoffbeladener Stents liegt in der Einstellung der Dosierung des freizusetzenden Wirkstoffs. Hierbei ist limitierend, dass die Menge des auf die Außenseite der Stents aufzubringenden Wirkstoffs stark begrenzt ist, da die für die Aufbringung bereitstehenden Flächen am Stent sehr klein sind. Bei Stents aus biokorrodierbaren Magnesiumlegierungen kann zusätzlich das Problem auftreten, dass das durch Korrosion des Werkstoffs entstehende stark alkalische Milieu das Resorptionsverhalten des aufzunehmenden Wirkstoffs negativ beeinflusst. So werden Wirkstoffe teils als Hydrochloride eingesetzt, wenn die Löslichkeit des Wirkstoffs sonst zu gering ist. Derartige Hydrochloride werden jedoch im entstehenden stark alkalischen Milieu wieder in die schlecht löslichen deprotonierten Wirkstoffe überführt.

### Zusammenfassung der Erfindung

Der vorliegenden Erfindung liegt die Aufgabe zugrunde, eines oder mehrere der zuvor geschilderten Probleme zu lösen oder zumindest zu mindern. Insbesondere soll die Resorption der Wirkstoffe verbessert werden, wenn sie Bestandteil einer Beschichtung oder Kavitätenfüllung eines Implantats aus einem biokorrodierbaren Werkstoff sind.

Diese Aufgabe wird erfindungsgemäß gelöst durch ein Implantat mit einem Grundkörper aus einem biokorrodierbaren Werkstoff und mit einer aktiven Beschichtung oder Füllung einer Kavität bestehend oder enthaltend die Komponenten
a) mindestens eine pharmazeutisch aktive Substanz; und
b) einen Hilfsstoff zur Verbesserung der Permeabilität der mindestens einen pharmazeutisch aktiven Substanz.

Unter einem Hilfsstoff zur Verbesserung der Permeabilität der mindestens einen pharmazeutisch aktiven Substanz (Wirkstoff) werden im Rahmen der vorliegenden Erfindung Verbindungen verstanden, die bedingt durch chemische, physikalische oder physiologische Wechselwirkungen die Penetration der pharmazeutisch aktiven Substanz durch das Gewebe zu erhöhen und somit die Gewebskonzentration an Wirkstoff steigern. Als Hilfsstoffe eignen sich beispielsweise aus Benzalkoniumchlorid, α-Tocopherol, Glucose, Lactose, Calciumphosphat, Calciumhydrogenphosphat, Natriumhydrogencarbonat, Natriumcarbonat, Titanoxid, Zinkoxid, Magnesiumoxid, Silikate wie hochdisperses Siliciumdioxid (Kolloidale Kieselsäure, Aerosil, Si02), Talkum, Kaolin, Bentonit, aliphatische Alkohole, DMSO, Glycerol, Propylenglykol, Stearinsäure, Zucker und Zuckeralkohole, Cyclodextrine wie α-Cyclodextrin, β-Cyclodextrin, γ-Cyclodextrin, sowie Mannitol, Sorbitol, Stärken, Cellulosepulver, Celluloseester und -ether wie Methylcellulose, Ethylcellulose, Hydroxyethylcellulose, Hydroxypropylcellulose, Hydroxypropylmethylcellulose, Natriumcarboxymethylcellulose, Celluloseacetatphthalat, Hydroxypropylmethylcelluloseacetatphthalat und mikrokristalline Cellulose, sowie Gelatine, Gummi arabicum, Pektin, Xanthan, Alginate, Schellack, Polyacrylsäuren wie Carbomere und Eudragit^{®}, sowie Polyvinylpyridin, Polyvinylpyrrolidon, Polyvinylalkohol, Polyvinylacetat, Mischungen aus Polyvinylpyridin und Polyvinylacetat, Polyethylenglykol, Vaselin, synthetische und natürliche Fette, Silikone, amphiphile oder oberflächenaktive Hilfsstoffe wie anionenaktive Tenside, Saponide; kationische Tenside wie Cetylpyridiniumchlorid, nichtionogene Tenside; Polyoxyethylensorbitan, Macrogolglycerolfettsäureester, Fettalkoholether des Polyoxyethylens, Fettsäureester der Saccharose und insbesondere D-α-Tocopheryl-1000-succinat, amphotere Tenside, sowie Komplexemulgatoren wie Cetylstearylalkohol (Typ A und B), quartäre Ammoniumverbindungen, Konservierungsmittel, Antioxidantien wie Citronensäure, Citraconsäure, Weinsäure, Mono- und Polyphosphate, organische Phosphate wie Dodecylphosphat, Hexosephosphat, sowie Butyryltrihexylcitrat (BTHC) und Triethylcitrat.

Besonders bevorzugte Hilfsstoffe sind Hyaluronidasen oder Hyaluronatlyasen, Derivat des Glycerins, insbesondere Glycerintrilaurat, -trimyristat, -tripalmiat und -tristerat, sowie polyglycolisierte Glyceride wie Gelucire^{®} 50/13, sowie Stoffe aus der Gruppe der Weichmacher wie Tricresylphosphat (TCP), Tributylphosphat (TBP), acetylierte Monoglyceride wie Alkylcitrat, Triethylcitrat, Acetyltriethylcitrat, Tributylcitrat, Acetyltributylcitrat, Trioctylcitrat, Acetyltrioctylcitrat, Trihexylcitrat, Acetyltrihexylcitrat, Butyryltrihexylcitrat, Trimethylcitrat. Diese zeichnen sich insbesondere durch eine deutlich verbesserte Gewebsaufnahme aus. Insbesondere bevorzugt sind Hyaluronidasen oder Hyaluronatlyasen.

Der Erfindung liegt die Erkenntnis zugrunde, dass in Gegenwart von Hyaluronidasen oder Hyaluronatlyasen das lokale Resorptionsverhalten von aktiven Substanzen insbesondere im sich bei Degradation des Stents entstehenden stark alkalischen Milieu verbessern lässt. Durch das alkalische Medium wird eine Vielzahl von neutralen aktiven Substanzen umgepolt. Diese geladenen Verbindungen können möglicherweise ihre Funktion nicht mehr übernehmen oder sind nicht mehr in der Lage, an ihren Zielort zu gelangen. Allerdings ermöglichen die erfindungsgemäßen Hilfsstoffe eine verbesserte Permeabilität dieser ggf. beeinträchtigten aktiven Substanzen. Von erheblicher Bedeutung ist zudem, dass die Wirkung von Hyaluronsäure-spaltenden Enzymen in Gegenwart von Magnesiumionen gesteigert ist.

Hyaluronsäure ist ein lineares saures Mucopolysaccharid, dessen strukturelle Grundeinheit auf einem Disaccharid basiert, das aus den Zuckern N-Acetylglukosamin und D-Glucuronsäure besteht, die miteinander durch β-1-3 glycosidische Bindung verknüpft sind. Über eine β-1-3 glycosidische Bindung sind diese Disaccharide ihrerseits wieder zu einem Polysaccharid untereinander verbunden. So entstehen basierend auf diesen Disaccharideinheiten Makromoleküle mit Molmassen zwischen 200 und 10 000 kDa. Auf Grund ihrer Struktur besitzt die Hyaluronsäure die Fähigkeit, eine Vielzahl von Wasserstoffbrückenbindungen auszubilden. Durch dieses hohe Hydratationsvermögen ist die Hyaluronsäure einer der Strukturkomponenten im Gewebe von Vertebraten, über die das Wassereinlagerungsvermögen und damit das osmotische Gleichgewicht gesteuert werden. Durch ihren Quellungsgrad kann sie auch als Barriere wirken, über die der Fluss von Ionen, Proteinen und Zellen im Gewebe beeinflusst wird.

Hyaluronsäure-spaltende Enzyme stellen auf Grund ihrer Vielfalt und Komplexität eine sehr umfangreiche Gruppe von Enzymen dar, die in der Natur in einer Vielzahl von Organismen vorkommen und hier zum Teil recht unterschiedliche Funktionen erfüllen. In Säugern beeinflussen Hyaluronidasen viele regulative Abläufe auf zellulärer Ebene. Die Hyaluronsäure-spaltenden Enzyme werden entsprechend ihres Spaltungsmechanismus, des Spaltortes und den resultierenden Spaltprodukten in drei große Hauptgruppen eingeteilt.

Hyaluronidasen umfassen Hyaluronatglucanohydrolasen und β-1-3 Hyaluronatglucanohydrolasen. Hyaluronatglucanohydrolasen spalten die Hyaluronsäure durch Hydrolyse als Endohydrolasen an der β-1-3 glykosidischen Bindung zu gesättigten Oligosacchariden mit D-Glucuronsäure am nichtreduzierten und N-Acetyl-β-D-glucosamin am reduzierten Molekülende. β-1-3 Hyaluronatglucanohydrolasen spalten Hyaluronsäure durch Hydrolyse als Endohydrolase an der D-Glucuronsäure in β-1-3 Stellung zu gesättigten Oligosacchariden mit N-Acetyl-β-D-glucosamin am nicht reduzierten und D-Glucuronsäure am reduzierten Molekülende.

Eine weitere Gruppe stellen Hyaluronatlyasen dar, die Hyaluronsäure durch β-Eliminierung an der β-1-4 glykosidischen Bindung am N-Acetyl-β-D-glukosamin zu Oligo- und Disacchariden mit ungesättigten Hexuronsäure-Resten am nichtreduzierten Molekülende spalten.

Die Eignung von Hyaluronsäure-spaltenden Enzymen zur Resorptions- und Penetrationsförderung ist bekannt. Um die Geschwindigkeit der Geweberesorption nach subkutaner oder intramuskulärer Applikation von pharmazeutischen Wirkstoffen zu beschleunigen oder die Resorption von Arzneimitteln durch die intakte Haut zu verbessern, werden Hyaluronidasen eingesetzt. Da die meisten der subkutan oder intramuskulär verabreichten Wirkstoffe über den Interzellularraum in die Kapillaren gelangen, spielt die Beschaffenheit der interzellulären Grundsubstanz, in der die Hyaluronsäure dominiert, eine wichtige Rolle. Wird die Hyaluronsäure durch die Hyaluronidasen abgebaut, kommt es zu einer beschleunigten Ausbreitung des Wirkstoffs im Interstitium und damit zur verbesserten Wirkstoffresorption. Sie fördert als "spreading factor" die Strukturauflockerung von Binde- und Stützgewebe und so den Flüssigkeitsaustausch zwischen den Geweben und dem Gefäßsystem. Die penetrationsfördernden Eigenschaften der Hyaluronidase werden vor allem in der lokalen und ophthalmologischen Anästhesie angewandt. Des Weiteren wird die Hyaluronidase bzw. Hyaluronatlyase in topischen Formulierungen zur Resorptionsbeschleunigung von Wirkstoffen durch die intakte Haut eingesetzt, da topisch applizierte Hyaluronidase in der Lage ist, das Stratum corneum zu durchdringen.

Bei Versuchen an hyperlipämischen Kaninchen zum Einfluss wiederholter Hyaluronidasegaben, konnte nachgewiesen werden, dass es unter der Wirkung von Hyaluronidase zu einer Erniedrigung des Blutcholesterin- und des Fibringehaltes, zur Erhöhung des Heparingehaltes im Plasma, zu einer gesteigerten vasculären Permeabilität im Gewebe und zu einer signifikanten Abnahme der atheromatösen Gefäßplaques kam. Deshalb wird die Behandlung von Gefäßkrankheiten des Menschen bei Herzarrhythmien, Thrombosen, zerebralen Infarkten, zerebralen Thrombosen und Herzinfarkten mit hochdosierter Hyaluronidase empfohlen. Diese Plaques-lösende Wirkung der aus Rindertestis gewonnenen Hyaluronidase konnte auch für die bakteriellen Hyaluronatlyase bestätigt werden. Neben der direkten Infarktbehandlung ist schon länger bekannt, dass durch Hyaluronidasen die Schäden reduziert werden können, die infolge eines Infarktes auftreten. So hat Hyaluronidase eine positive Wirkung auf den kollateralen Blutfluss in das ischämische Gewebe. Hyaluronidase erhöht das Herz-Lymph-Volumen und verbessert dadurch die postischämische Genesung der Herzfunktion. Durch aktive Drainage der Herzlymphe mit Hyaluronidase wird die Bildung myokardialer Ödeme gemildert und dadurch das Herz in seiner Funktion geschützt. Die vom Myokard freigesetzten Proteine akkumulieren signifikant in der Herzlymphe, deren Volumen durch das Infarktereignis graduell zunimmt. Durch Hyaluronidase kann dieser Lymphfluss erhöht werden, wodurch die Okklusion bzw. der Kollaps der Lymphgefäße verhindert wird. Unterstützt werden könnte dieser Prozess auch durch die Hyaluronidase stimulierte endogene Freisetzung von NO, wodurch die Thrombozyten und Leukozyten-Adhäsion und -Aggregation verhindert wird. Hyaluronidasen haben damit für sich genommen bereits einen positiven pharmazeutischen Effekt.

Die aktive Beschichtung bzw. Kavitätenfüllung beinhaltet neben dem Hilfsstoff zur Verbesserung der Permeabilität der mindestens einen pharmazeutisch aktiven Substanz, insbesondere dem Hyaluronsäure-spaltenden Enzym wenigstens eine pharmazeutisch aktive Substanz. Insbesondere ist diese pharmazeutisch aktive Substanz ausgewählt aus der Gruppe umfassend Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Myolimus, Novolimus, Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und I-loprost; Statinen, vorzugsweise Simvastatin, Mevastatin, Atorvastatin, Lovastatin, Pitavastatin, Pravastatin und Fluvastatin; Estrogenen, vorzugsweise 17b-Estradiol, Daizein und Genistein; Lipidregulatoren, vorzugsweise Fibrate; Immunsuppressiva; Vasodilatatoren, vorzugsweise Sartane; Calciumkanalblocker; Calcineurininhibitoren, vorzugsweise Tacrolimus; Antiinflammatorika, vorzugsweise Imidazole; Antiallergika; Oligonucleotiden, vorzugsweise Decoy-Oligodesoxynukleotid (dODN); Endothelbildner, vorzugsweise Fibrin; Steroiden; Proteinen/Peptiden; Proliferationshemmer; Analgetika und Antirheumatika; Endothelinrezeptor-Antagonisten, vorzugsweise Bosentan; Rho-Kinase Inhibitoren, vorzugsweise Fasudil; RGD-Peptiden und zyklische RGD (cRGD) (umfassend die Sequenz Arg-Gly-Asp); und organischen Gold- oder Platinverbindungen.

Der Hilfsstoff zur Verbesserung der Permeabilität der mindestens einen pharmazeutisch aktiven Substanz, insbesondere das Hyaluronsäure-spaltende Enzym, und die pharmazeutisch aktive Substanz können ferner in eine Matrix, vorzugsweise aus einem biokorrodierbaren Polymer, eingebettet sein. Biodegradierbare Polymere umfassen insbesondere Polydioxanon; Polyorthoester; Polyesteramide; Polycaprolacton, Polyglycoliden; Polylactiden, vorzugsweise Poly(L-lactid), Poly(D-lactid), Poly(D,L-lactid) sowie Blends, Co-Polymere und Tri-Polymere hiervon, vorzugsweise Poly(L-lactid-co-glycolid), Poly(D-L-lactid-coglycolid), Poly(L-lactid-co-L-lactid), Poly(L-lactid-co-trimethylencarbonat); Polysaccharide, vorzugsweise Chitosan, Levan, Hyaluronsäure, Heparin, Dextran, Chodroitinsulfat und Cellulosen; Polyhydroxyvalerat; Ethylvinylacetat; Polyethylenoxiden; Polyphosphorylcholin; Fibrin; Albumin; und/oder Polyhydroxybuttersäuren, vorzugsweise ataktische, isotaktische und/oder syndiotaktische Polyhydroxybuttersäure sowie deren Blends. Ebenso nicht oder langsam degradierende Polymere wie: Polyphoshazene wie die Polyaminophoshazene oder Poly[bis(trifluroexthoxy)phosphazen], Polyurethane, wie Pellethan, oder Polyether und Polyetherblockamide, wie Pebax, und Polyamide.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Der Stent kann vor oder nach dem Crimpen auf einen Ballon beschichtet werden.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Biokorrodierbare metallische Werkstoffe im Sinne der Erfindung umfassen Metalle und Legierungen ausgewählt aus der Gruppe umfassend Eisen, Wolfram, Zink, Molybdän und Magnesium und insbesondere solche biokorrodierbare metallische Werkstoffe, die in wässriger Lösung zu einem alkalischen Produkt korrodieren.

Beispielsweise besteht der metallische Grundkörper aus Reineisen, einer biokorrodierbaren Eisenlegierung, einer biokorrodierbaren Wolframlegierung, einer biokorrodierbaren Zinklegierung oder einer biokorrodierbaren Molybdänlegierung. Vorzugsweise besteht der metallische Grundkörper aus Magnesium. Insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist in ihrer Zusammensetzung so zu wählen, dass sie biokorrodierbar ist. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, M_{g}SO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Eine Beschichtung im Sinne der Erfindung ist eine zumindest abschnittsweise Auftragung der Komponenten auf den Grundkörper des Stents. Vorzugsweise wird die gesamte Oberfläche des Grundkörpers des Stents von der Beschichtung bedeckt. Eine Schichtdicke liegt vorzugsweise im Bereich von 1 µm bis 100 µm, vorzugsweise besonders bevorzugt 3 µm bis 15 µm. Die Beschichtung besteht aus zumindest einem Wirkstoff und dem Hilfsstoff zur Verbesserung der Permeabilität der mindestens einen pharmazeutisch aktiven Substanz, insbesondere dem Enzym (Hyaluronidase oder Hyaluronatlyase). Die Beschichtung kann weiterhin eine die beiden Komponenten aufnehmende Matrix, insbesondere aus einem biokorrodierbaren Polymer, beinhalten. Alternativ können die genannten Komponenten Bestandteil einer Kavitätenfüllung sein. Die Kavität befindet sich an der Oberfläche oder im Innern des Grundkörpers, so dass im letzteren Fall die Freisetzung der Komponenten erst nach Freilegung der Kavität durch Degradation des Grundkörpers erfolgt. Wirkstoff und Hilfsstoff können räumlich voneinander getrennt, ggf. auch in verschiedenen Matrices, in der Beschichtung vorliegen.

In einer bevorzugten Ausführungsform liegt der Hilfsstoff zur Verbesserung der Permeabilität der mindestens einen pharmazeutisch aktiven Substanz, insbesondere Hyaluronidase oder Hyaluronatlyase, in einer Konzentration in der Größenordnung eines Additivs, zwischen 0,01 und 2% in der Beschichtung vor.

Eine Zugabe in der Größenordnung eines Additivs verbessert die Gewebsaufnahme ohne die sonstigen mechanischen Eigenschaften des Implantats nachteilig zu beeinflussen. Die Zugabe in oben beschreibender Menge verändert bei vielen Wirkstoffen, wie z.B. Paclitaxel, die Kristallinität in so geeigneter Form, dass die Bioverfügbarkeit über das leichtere und schnellere Lösungsvermögen bei geänderter Kristallinität verbessert wird.

Die Erfindung wird nachfolgend anhand eines Ausführungsbeispiels näher erläutert.

### Ausführungsbeispiel 1 - Beschichtung eines Stents

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) werden mit Chloroform gewaschen, mit deionisiertem Wasser gespült und getrocknet.

Es werden folgende Beschichtungslösungen angesetzt:

| | |
|---|---|
| Lösung A | Lösungsmittel Chloroform PLLA (Handelsname L210 von Boehringer Ingelheim) 1 g/l Paclitaxel 150 mg/l |
| Lösung B | Lösungsmittel 100 Gewichtsteile Chloroform und Gewichtsteile Methanol PLLA (Handelsname L210 von Boehringer Ingelheim) 1 g/l Hyaluronatlyase (25.000 - 50.000 IU). |

Die Lösungen werden schichtweise auf den Stent durch Aufsprühen aufgetragen und getrocknet. Die letzte Schicht wird mit Lösung B aufgetragen.

## Patentansprüche

1. Implantat mit einem Grundkörper aus einem biokorrodierbaren Werkstoff und mit einer aktiven Beschichtung oder Füllung einer Kavität bestehend aus oder enthaltend die Komponenten
a) mindestens eine pharmazeutisch aktive Substanz; und
b) einen Hilfsstoff zur Verbesserung der Permeabilität der mindestens einen pharmazeutisch aktiven Substanz.

2. Implantat nach Anspruch 1, bei dem das Implantat ein Stent ist.

3. Implantat nach Anspruch 1 oder 2, bei dem der biokorrodierbare Werkstoff aus Magnesium oder einer Magnesiumlegierung besteht.

4. Implantat nach einem der vorherigen Ansprüche, bei dem der Hilfsstoff ausgewählt ist aus der Gruppe bestehend aus Benzalkoniumchlorid, α-Tocopherol, Glucose, Lactose, Calciumphosphat, Calciumhydrogenphosphat, Natriumhydrogencarbonat, Natriumcarbonat, Titanoxid, Zinkoxid, Magnesiumoxid, Silikate wie hochdisperses Siliciumdioxid (Kolloidale Kieselsäure, Aerosil, Si02), Talkum, Kaolin, Bentonit, aliphatische Alkohole, DMSO, Glycerol, Propylenglykol, Stearinsäure, Zucker und Zuckeralkohole, Cyclodextrine wie α-Cyclodextrin, β-Cyclodextrin, γ-Cyclodextrin, sowie Mannitol, Sorbitol, Stärken, Cellulosepulver, Celluloseester und -ether wie Methylcellulose, Ethylcellulose, Hydroxyethylcellulose, Hydroxypropylcellulose, Hydroxypropylmethylcellulose, Natriumcarboxymethylcellulose, Celluloseacetatphthalat, Hydroxypropylmethylcelluloseacetatphthalat und mikrokristalline Cellulose, sowie Gelatine, Gummi arabicum, Pektin, Xanthan, Alginate, Schellack, Polyacrylsäuren wie Carbomere, sowie Polyvinylpyridin, Polyvinylpyrrolidon, Polyvinylalkohol, Polyvinylacetat, Mischungen aus Polyvinylpyridin und Polyvinylacetat, Polyethylenglykol, Vaselin, synthetische und natürliche Fette, Silikone, amphiphile oder oberflächenaktive Hilfsstoffe wie anionenaktive Tenside, Saponide; kationische Tenside wie Cetylpyridiniumchlorid, nichtionogene Tenside; Polyoxyethylensorbitan, Macrogolglycerolfettsäureester, Fettalkoholether des Polyoxyethylens, Fettsäureester der Saccharose und insbesondere D-α-Tocopheryl-1000-succinat, amphotere Tenside, sowie Komplexemulgatoren wie Cetylstearylalkohol (Typ A und B), quartäre Ammoniumverbindungen, Konservierungsmittel, Antioxidantien wie Citronensäure, Citraconsäure, Weinsäure, Mono- und Polyphosphate, organische Phosphate wie Dodecylphosphat, Hexosephosphat, Hyaluronidasen oder Hyaluronatlyasen, Derivate des Glycerins wie Glycerintrilaurat, - trimyristat, -tripalmiat und -tristerat, sowie polyglycolisierte Glyceride, sowie Stoffe aus der Gruppe der Weichmacher wie Tricresylphosphat (TCP), Tributylphosphat (TBP), acetylierte Monoglyceride wie Alkylcitrat, Triethylcitrat, Acetyltriethylcitrat, Tributylcitrat, Acetyltributylcitrat, Trioctylcitrat, Acetyltrioctylcitrat, Trihexylcitrat, Acetyltrihexylcitrat, Butyryltrihexylcitrat und Trimethylcitrat.

5. Implantat nach Anspruch 4, bei dem der Hilfsstoff Hyaluronidasen oder Hyaluronatlyasen ist.

6. Implantat nach einem der vorherigen Ansprüche, bei dem die Komponenten a) und b) in eine Matrix aus einem biokorrodierbaren Polymer eingebettet sind.

7. Implantat nach einem der vorherigen Ansprüche, bei dem die pharmazeutisch aktive Substanz ausgewählt ist aus der Gruppe umfassend Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Myolimus, Novolimus, Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und I-loprost; Statinen, vorzugsweise Simvastatin, Mevastatin, Atorvastatin, Lovastatin, Pitavastatin, Pravastatin und Fluvastatin; Estrogenen, vorzugsweise 17b-Estradiol, Daizein und Genistein; Lipidregulatoren, vorzugsweise Fibrate; Immunsuppressiva; Vasodilatatoren, vorzugsweise Sartane; Calciumkanalblocker; Calcineurininhibitoren, vorzugsweise Tacrolimus; Antiinflammatorika, vorzugsweise Imidazole; Antiallergika; Oligonucleotiden, vorzugsweise Decoy-Oligodesoxynukleotid (dODN); Endothelbildner, vorzugsweise Fibrin; Steroiden; Proteinen/Peptiden; Proliferationshemmer; Analgetika und Antirheumatika; Endothelinrezeptor-Antagonisten, vorzugsweise Bosentan; Rho-Kinase Inhibitoren, vorzugsweise Fasudil; RGD-Peptiden und zyclische RGD (cRGD) (umfassend die Sequenz Arg-Gly-Asp); und organischen Gold- oder Platinverbindungen.

8. Implantat nach einem der vorherigen Ansprüche, wobei die Komponente b) in einer Konzentration zwischen 0,01 und 2% in der Beschichtung oder Füllung einer Kavität vorliegt.
